# EUROPEAN PATENT APPLICATION

(11) **EP 3 097 884 A1**
(43) Date of publication of application: **30.11.2016**
(21) Application number: 15740876.6
(22) Date of filing: 14.01.2015
(51) Int. Cl.: A61B 90/00, B25J 17/02

(54) **SURGICAL TOOL AND MEDICAL MANIPULATOR SYSTEM**

(30) Priority: 23.01.2014 JP 2014010543
(71) Applicant: Olympus Corporation, Tokyo 192-8507 (JP)
(72) Inventor: HYODO, Ryoji, Hachioji-shi, Tokyo 192-8507 (JP); KISHI, Kosuke, Hachioji-shi, Tokyo 192-8507 (JP)
(74) Representative: Schicker, Silvia
(86) International application number: PCT/JP2015/050738
(87) International publication number: WO 2015/111475

(57) **Abstract**

The surgical instrument includes a long member, a link portion, a treatment portion, a drive control unit, and a power transmission member. The link portion includes a first rotary shaft having a first rotation axis perpendicular to the longitudinal axis at a distal end of the long member and a second rotary shaft that is movable with respect to the first rotation axis using the first rotation axis as a center of rotational movement, that has a second rotation axis that extends in parallel with the first rotation axis, and that is linked to the shaft. The power transmission member is fixed to a part of an outer circumference of the shaft such that the first path and the second path face opposite directions in a circumferential direction of the shaft, and when viewed in a direction in which the first rotation axis and the second rotation axis extend, the first path and the second path intersect at a point between the first rotation axis and the second rotation axis.

## Description

### [Technical Field]

The present invention relates to a surgical instrument and a medical manipulator system. Priority is claimed on Japanese Patent Application No. 2014-010543, filed January 23, 2014, the content of which is incorporated herein by reference.

### [Background Art]

In the related art, a medical manipulator having a bendable joint at a distal end portion is known (for example, refer to Patent Literature 1). The manipulator described in Patent Literature 1 includes a wire that opens and closes an openable and closable grasper, and a joint configured to swing the grasper. The wire that opens and closes the grasper is inserted into the joint along a path in which a path length of the wire is not changed when the grasper is swung.

### [Citation List]

### [Patent Literature]

### [Patent Literature 1]

Japanese Patent No. 3912251

### [Summary of Invention]

### [Technical Problem]

In the manipulator described in Patent Literature 1, when the grasper is opened and closed using the wire, tension is applied to the wire to transmit appropriate force to the grasper. However, in the technique disclosed in Patent Literature 1, when tension is applied to the wire in order to open and close the grasper, a force bending the joint is applied to the joint configured to swing the grasper. Due to the force, in order to assemble the manipulator to which appropriate tension is applied, complicated adjustment is necessary.

The present invention has been made in view of the above-described situation, and an object of the invention is to provide a surgical instrument and a medical manipulator system that are easily assembled.

### [Solution to Problem]

A surgical instrument according to a first aspect of the present invention includes: a long member having a longitudinal axis; a link portion that is disposed at a distal end of the long member and bendable with respect to the long member in a direction perpendicular to the longitudinal axis; a treatment portion that is disposed at a distal end of the link portion, includes a shaft that is rotatable with respect to the link portion, and performs treatment on a treatment target region; a drive control unit disposed at a proximal end of the long member to perform an operation of rotating the shaft with respect to the link portion; and a power transmission member that is connected to the shaft and the drive control unit and includes a first path and a second path through which power is transmitted from the drive control unit to the shaft, wherein the link portion includes a first rotary shaft having a first rotation axis perpendicular to the longitudinal axis at the distal end of the long member, and a second rotary shaft that is movable with respect to the first rotation axis using the first rotation axis as a center of rotational movement, that has a second rotation axis that extends in parallel with the first rotation axis, and that is linked to the shaft, and wherein, the power transmission member is fixed to a part of an outer circumference of the shaft such that the first path and the second path face opposite directions in a circumferential direction of the shaft, and when viewed in a direction in which the first rotation axis and the second rotation axis extend, the first path and the second path intersect at a point between the first rotation axis and the second rotation axis.

According to a second aspect of the present invention, in the surgical instrument according to the first aspect, when viewed in a direction perpendicular to the longitudinal axis and perpendicular to the first rotation axis and the second rotation axis, the first path and the second path may be spaced from each other in a direction in which the first rotation axis and the second rotation axis extend, in at least a section from a center of rotation of the shaft to the first rotation axis.

According to a third aspect of the present invention, in the surgical instrument according to the second aspect, the shaft may include a first pulley at which the first path of the power transmission member is disposed, and a second pulley at which the second path of the power transmission member is disposed, wherein the first pulley and the second pulley may be spaced from each other in a direction in which the center of rotation of the shaft extends; and wherein, when viewed in a direction of the second rotation axis, in a section from the center of rotation of the shaft to the second rotation axis, the first path and the second path may be substantially parallel.

According to a fourth aspect of the present invention, in the surgical instrument according to the first aspect, the power transmission member may include a first end and a second end fixed to the shaft, and an intermediate part disposed at the drive control unit, and wherein the drive control unit may include a moving body linked to the intermediate part, and a tension adjusting unit capable of applying tension to the power transmission member by moving the moving body.

A medical manipulator system according to a fifth aspect of the present invention includes the surgical instrument according to the above aspect and an arm attached to the surgical instrument and configured to move the surgical instrument.

### [Advantageous Effects of Invention]

A surgical instrument and a medical manipulator system of the present invention are easily assembled.

### [Brief Description of Drawings]

Fig. 1 is an overall view of a medical manipulator system including a surgical instrument according to a first embodiment of the present invention.
Fig. 2 is a diagram schematically showing the surgical instrument according to the first embodiment of the present invention.
Fig. 3 is a right side view schematically showing a part of the surgical instrument according to the first embodiment of the present invention.
Fig. 4 is a left side view schematically showing a part of the surgical instrument according to the first embodiment of the present invention.
Fig. 5 is a front view of the surgical instrument according to the first embodiment of the present invention.
Fig. 6 is a diagram schematically showing a configuration of a modification of the first embodiment of the present invention.
Fig. 7 is a right side view schematically showing a part of a surgical instrument of the modification of the first embodiment of the present invention.

### [Description of Embodiments]

### (First embodiment)

A surgical instrument 1 according to a first embodiment of the present invention will be described.

The surgical instrument 1 according to the present embodiment is integrated into a medical manipulator system configured to perform a medical procedure.

First, a configuration of a medical manipulator system 1000 into which the surgical instrument 1 according to the present embodiment is integrated will be described.

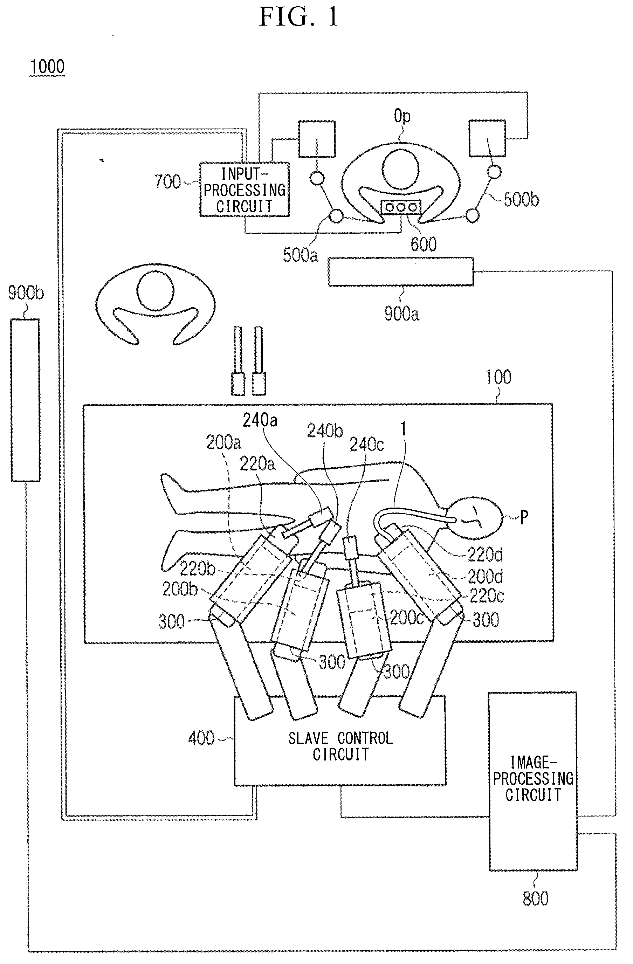

Fig. 1 is a diagram schematically showing an exemplary configuration of the medical manipulator system 1000 to which the surgical instrument of the present invention is applied.

Fig. 1 shows an exemplary master-slave type medical manipulator system. The master-slave type medical manipulator system is a system that includes two types of arms, a master arm and a slave arm, and controls the slave arm remotely to follow a movement of the master arm. In the present embodiment, the surgical instrument 1 is attachable to the slave arm.

The medical manipulator system 1000 shown in Fig. 1 includes an operating table 100, slave arms 200a, 200b, 200c, and 200d, a slave control circuit 400, master arms 500a and 500b, an operation unit 600, an input processing circuit 700, an image processing circuit 800, an operator display 900a, and an assistant display 900b.

Hereinafter, for simplicity of description, reference symbols "Xa, Xb, ..., Xz" in alphabetical order are denoted as "Xa to Xz" in some cases. For example, "the slave arms 200a, 200b, 200c, and 200d" may be denoted as "the slave arms 200a to 200d."

The operating table 100 is a table on which a patient P, who is an observation and treatment target, lies. In the vicinity of the operating table 100, slave arms 200a to 200d are installed. The slave arms 200a to 200d may be installed at the operating table 100.

Each of the slave arms 200a to 200d includes a plurality of joints with multiple degrees of freedom. The slave arms 200a to 200d determine positions of the surgical instrument 1, surgical instruments 240a to 240c, and the like that are mounted on distal end sides (sides toward a body cavity of the patient P) of the slave arms 200a to 200d with respect to the patient P lying on the operating table 100, by bending the joints with multiple degrees of freedom. The joints with multiple degrees of freedom are separately driven by a power unit (not shown). As the power unit, a motor having a servomechanism including, for example, an incremental encoder and a decelerator can be used. A movement control of the power unit is performed by the slave control circuit 400.

The surgical instrument 1 and the other surgical instruments 240a to 240c may be rigid or flexible. That is, as the surgical instrument 1 and the other surgical instruments 240a to 240c, an instrument in which an operating body configured to perform treatment on a living body is moved by pushing and pulling a rigid rod and an instrument in which an operating body configured to perform treatment on a living body is moved by pulling a flexible wire can be appropriately selected and used. Even when the surgical instrument 1 and the other surgical instruments 240a to 240c are rigid, a configuration in which the operating body is moved by pulling the flexible wire may be provided. In the present embodiment, the surgical instrument 1 has a configuration in which a driving force moving the operating body is transmitted to the operating body through the flexible wire.

In Fig. 1, for example, the surgical instruments 240a to 240c to be inserted into the abdominal cavity of the patient are rigid. The surgical instrument 1 that is introduced into the patient's body through, for example, a natural orifice such as the mouth and then through the gastrointestinal tract, is flexible.

The slave control circuit 400 includes, for example, a CPU and a memory. The slave control circuit 400 stores a predetermined program configured to control the slave arms 200a to 200d, and controls movements of the slave arms 200a to 200d or the surgical instrument 1 and the other surgical instruments 240a to 240c according to a control signal from the input processing circuit 700. That is, the slave control circuit 400 specifies a slave arm (or the surgical instrument 1) that is an operation target of the master arm operated by an operator Op based on a control signal from the input processing circuit 700, and calculates the amount of driving necessary to move the specified slave arm or the like according to an amount of operation of the master arm operated by the operator Op.

Then, the slave control circuit 400 controls the movement of the slave arm or the like that is an operation target of the master arm according to the calculated amount of driving. In this case, the slave control circuit 400 inputs a drive signal to a corresponding slave arm, and controls the magnitude or the polarity of a drive signal such that the amount of driving of the slave arm serving as the operation target becomes a target amount of driving according to a detection signal input from a position detector of the power unit depending on the movement of the corresponding slave arm.

The master arms 500a and 500b have a plurality of link mechanisms. In links of the link mechanism, a position detector, for example, an incremental encoder, is provided. By detecting the movement of each of the links using the position detector, the amount of operation of the master arms 500a and 500b is detected in the input processing circuit 700.

In the medical manipulator system 1000 shown in Fig. 1, the two master arms 500a and 500b are used to operate four slave arms, and there is a need to appropriately switch the slave arm serving as the operation target of the master arm. Such switching is performed by, for example, an operation of the operation unit 600 by the operator Op. It is needless to say that such switching is unnecessary when the number of master arms is the same as the number of slave arms and the operation targets are in one-to-one correspondence.

The operation unit 600 includes various operation members, for example, a switching button for switching the slave arm serving as the operation target of the master arms 500a and 500b, a scaling changing switch for changing a movement ratio between a master and a slave, and a foot switch configured to stop the system in an emergency. When any operation member of the operation unit 600 is operated by the operator Op, an operation signal according to the operation of the corresponding operation member is input from the operation unit 600 to the input processing circuit 700.

The input processing circuit 700 analyzes an operation signal from the master arms 500a and 500b and an operation signal from the operation unit 600, generates a control signal configured to control the medical manipulator system 1000 according to an analysis result of the operation signal, and inputs the signal to the slave control circuit 400.

The image processing circuit 800 performs various types of image processing configured to display an image signal input from the slave control circuit 400 and generates display image data in the operator display 900a and the assistant display 900b. The operator display 900a and the assistant display 900b are configured of, for example, liquid crystal displays, and display an image based on image data generated in the image processing circuit 800 according to an image signal acquired through an observation instrument.

In the medical manipulator system 1000 configured as described above, when the operator Op operates the master arms 500a and 500b, the corresponding slave arm and the surgical instrument 1 and the other surgical instruments 240a to 240c attached to the slave arm move according to a movement of the master arms 500a and 500b. Therefore, it is possible to perform a desired procedure on the patient P.

Note that reference symbols 220a, 220b, 220c, and 220d in Fig. 1 indicate surgical power transmission adapters. The surgical power transmission adapters 220a, 220b, and 220c connect the slave arms 200a, 200b, and 200c and the rigid surgical instruments 240a, 240b, and 240c, respectively. The surgical power transmission adapter 220d connects together the slave arm 200d and the flexible surgical instrument 1.

In addition, in the present embodiment, in the medical manipulator system 1000, a drape 300 for separating a portion on which sterilization is performed (a clean area) and a portion on which no sterilization is performed (an unclean area) is attached to the medical manipulator system 1000.

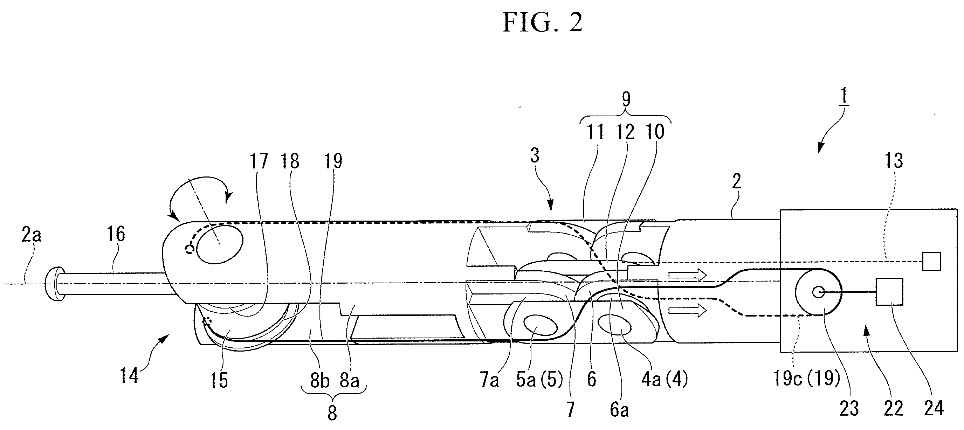

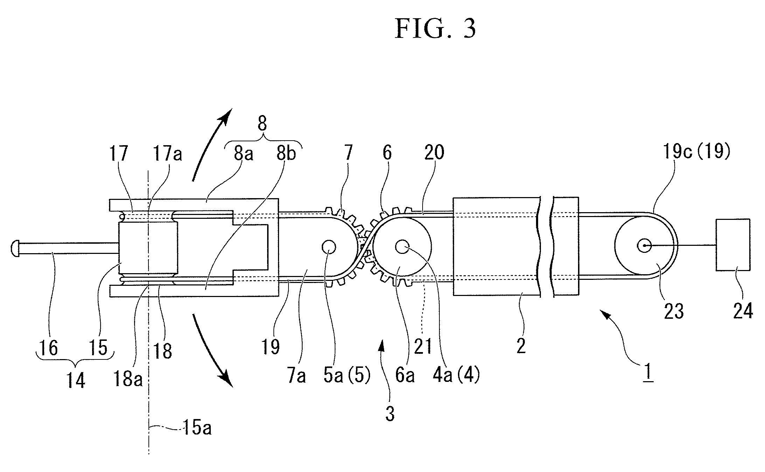

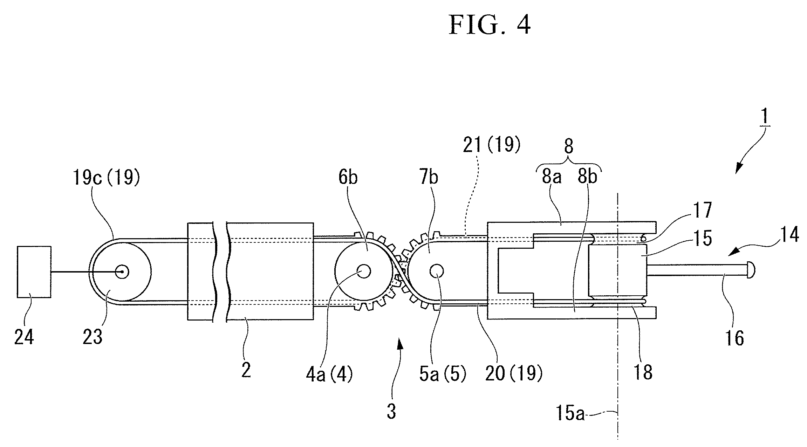

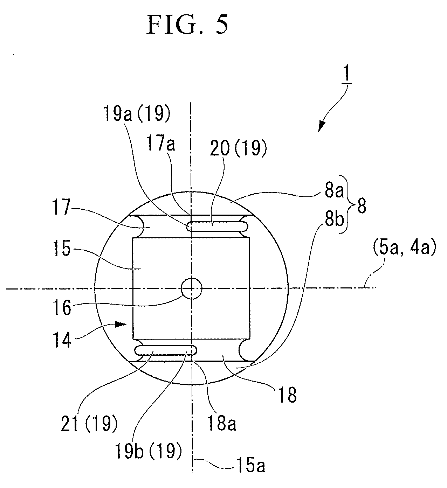

Next, the surgical instrument 1 integrated into the medical manipulator system 1000 will be described. Note that, in description of the surgical instrument 1 according to the present embodiment, a side toward the body cavity of the patient P while the surgical instrument 1 is integrated into the medical manipulator system 1000 is referred to as a distal side of the surgical instrument 1, and a side of a portion connected to the medical manipulator system 1000 at a side away from the patient P in the surgical instrument 1 is referred to as a proximal side of the surgical instrument 1. Fig. 2 is a diagram schematically showing the surgical instrument 1 according to the present embodiment. Fig. 3 is a right side view schematically showing a part of the surgical instrument 1. Fig. 4 is a left side view schematically showing a part of the surgical instrument 1. Fig. 5 is a front view of the surgical instrument 1.

The surgical instrument 1 according to the present embodiment is attached to the slave arms 200a to 200d shown in Fig. 1. The surgical instrument 1 moves according to an operation input to the master arms 500a and 500b.

The surgical instrument 1 shown in Fig. 2 is a medical instrument configured to perform treatment on a treatment target region. As shown in Fig. 2, the surgical instrument 1 includes a long member 2, a link portion 3, a treatment portion 14, and a drive control unit 22.

The long member 2 is a tubular member having a longitudinal axis 2a. The long member 2 may be flexible or rigid depending on a configuration of the slave arm 200d (refer to Fig. 1) to be attached. In the present embodiment, the long member 2 is flexible.

The link portion 3 includes a first rotary shaft 4 having a first rotation axis 4a perpendicular to the longitudinal axis 2a of the long member 2 at a distal end of the long member 2 and a second rotary shaft 5 that is movable with respect to the first rotation axis 4a using the first rotation axis 4a of the first rotary shaft 4 as a center of rotational movement and has a second rotation axis 5a.

Specifically, as shown in Figs. 2 to 4, the link portion 3 in the present embodiment includes a first engagement part 6 fixed to the distal end of the long member 2, the first rotary shaft 4 linked to the distal end of the long member 2 to pass through a center of the first engagement part 6, a support portion 8 linked to the treatment portion 14 including a second engagement part 7 engaged with the first engagement part 6, the second rotary shaft 5 that extends in parallel with the first rotary shaft 4 through a center of the second engagement part 7, and a link main body 9 that links the first rotary shaft 4 and the second rotary shaft 5.

The first engagement part 6 includes a gear tooth along a circumference whose center is a center of rotational movement of the first rotary shaft 4. The second engagement part 7 includes a gear tooth along a circumference whose center is a center of rotational movement of the second rotary shaft 5 and is engaged with the first engagement part 6. The first engagement part 6 and the second engagement part 7 include the teeth along the circumference having the same radius and the ratio thereof is set to have a relation of 1:1.

A proximal first circumferential portion 6a and a proximal second circumferential portion 6b, which form a disk shape whose center is a center of rotational movement of the first rotary shaft 4, are linked to the first engagement part 6.

The second engagement part 7 can move while rotating along a circumference of the first engagement part 6. Instead of the first engagement part 6 and the second engagement part 7, plate members that are relatively rotatable while circumferential portions are in contact with each other due to a frictional force may be provided.

In the present embodiment, while a configuration in which the first engagement part 6 and the second engagement part 7 are in frictional contact with each other when gears are engaged is shown, the present invention is not necessarily limited to the configuration in which gears are engaged in frictional contact. For example, instead of frictional contact according to engagement of gears, a mechanism in which two rotation bodies do not slip but can roll to rotate may be used, such as a configuration in which two rubber rollers (having a great frictional force) are brought in frictional contact without engagement of gears.

A distal first circumferential portion 7a and a distal second circumferential portion 7b, which form a disk shape whose center is a center of rotational movement of the second rotary shaft 5, are linked to the second engagement part 7.

The first rotary shaft 4 and the second rotary shaft 5 include axes (the first rotation axis 4a and the second rotation axis 5a) whose centers of rotational movement extend in a direction perpendicular to an extended line of the longitudinal axis 2a of the long member 2. The first rotation axis 4a and the second rotation axis 5a are parallel to each other.

The support portion 8 includes the second engagement part 7 at a proximal side. In addition, at a distal side of the support portion 8, a pair of supporting pieces 8a and 8b spaced apart from each other are formed. Each of the pair of supporting pieces 8a and 8bis linked to a support shaft 15 (to be described below) in the treatment portion 14 such that the pair of supporting pieces 8a and 8b are connected.

In the present embodiment, the support portion 8 supports the second rotation axis 5a and the support shaft 15 such that a direction in which a center of rotation 15a of the support shaft 15 extends and a direction in which the second rotation axis 5a extends form a right angle. More accurately, the support portion 8 supports the second rotation axis 5a and the support shaft 15 such that the center of rotation 15a of the support shaft 15 and the second rotation axis 5a are skew lines and a directional vector in which the center of rotation 15a of the support shaft 15 extends and a directional vector in which the second rotation axis 5a extends are perpendicular to each other. That is, in the present embodiment, the second rotation axis 5a and the support shaft 15 are linked through the support portion 8.

The link main body 9 maintains the distance between the first engagement part 6 and the second engagement part 7 to be constant such that the first engagement part 6 and the second engagement part 7 are in an engaged state. The link main body 9 includes a pair of outer members 10 and 11 that link both ends of the first rotary shaft 4 and the second rotary shaft 5, and a bending plate 12, which is a plate member into which the first rotary shaft 4 and the second rotary shaft 5 are inserted.

The bending plate 12 is rotatable about the first rotation axis 4a according to a pulling direction of a bending operation wire 13. Further, the bending plate 12 that rotates about the first rotation axis 4a moves the second rotary shaft 5 to be turned around the first rotary shaft 4 serving as a turning center.

By a pulling operation of the bending operation wire 13, the link portion 3 bends in a direction perpendicular to the longitudinal axis 2a of the long member 2 as a whole.

The treatment portion 14 is a member configured to perform treatment on a treatment target region. The treatment portion 14 includes the support shaft (shaft) 15 that is disposed at a distal end of the support portion 8 in the link portion 3 and is rotatable with respect to the support portion 8 and a treatment piece 16 that extends in a direction intersecting the center of rotation 15a of the support shaft 15 and fixed to the support shaft 15.

The support shaft 15 includes a pair of pulleys (a first treatment pulley 17 and a second treatment pulley 18) having the same diameter at a position spaced apart from each other with the treatment piece 16 interposed therebetween in a direction in which the center of rotation 15a of the support shaft 15 extends. The distance between the first treatment pulley (first pulley) 17 and the second treatment pulley (second pulley) 18 is substantially equal to diameters of the distal first circumferential portion 7a and the distal second circumferential portion 7b provided in the link portion 3. Therefore, when viewed in the direction in which the second rotation axis 5a extends, a power transmission member 19 extends in parallel in a section from the center of rotation 15a of the support shaft 15 to the second rotation axis 5a.

Further, when viewed in the direction in which the second rotation axis 5a extends, the second rotation axis 5a, a center 17a of the first treatment pulley 17, and a center 18a of the second treatment pulley 18 have a positional relation of vertices of an isosceles triangle with a line segment connecting the center 17a of the first treatment pulley 17 and the center 18a of the second treatment pulley 18 as a base.

In the present embodiment, when the link portion 3 is not bended but is linear with respect to the long member 2, the first treatment pulley 17 and the second treatment pulley 18 are equidistant from the longitudinal axis 2a of the long member 2.

In addition, diameters of the first treatment pulley 17 and the second treatment pulley 18 are substantially equal to the distance between the distal first circumferential portion 7a and the distal second circumferential portion 7b in the direction in which the second rotation axis 5a extends. Therefore, when viewed in the direction in which the center of rotation 15a of the support shaft 15 extends, the power transmission member 19 extends in parallel in the section from the center of rotation 15a of the support shaft 15 to the second rotation axis 5a.

When the link portion 3 is not bended but is linear with respect to the long member 2, the power transmission member 19 viewed in a direction of the longitudinal axis 2a of the long member 2 is disposed at a position that is rotationally symmetrical with respect to the longitudinal axis 2a of the long member 2 in the section from the center of rotation 15a of the support shaft 15 to the second rotation axis 5a.

A distal portion in the power transmission member 19 is wound on the first treatment pulley 17 and the second treatment pulley 18, and a distal side end in the power transmission member 19 is fixed to the first treatment pulley 17 and the second treatment pulley 18. A first path 20 of the power transmission member 19 (to be described below) is disposed in the first treatment pulley 17. A second path 21 of the power transmission member 19 (to be described below) is disposed in the second treatment pulley 18.

The power transmission member 19 is a linear member that includes a first end 19a fixed to the first treatment pulley 17 of the support shaft 15, a second end 19b fixed to the second treatment pulley 18 of the support shaft 15, and an intermediate part 19c hung on a driving pulley 23 of the drive control unit 22 (to be described below).

The power transmission member 19 includes the first path 20 and the second path 21 through which power is transmitted from the drive control unit 22 to the support shaft 15. The power transmission member 19 is wound on the first treatment pulley 17 and the second treatment pulley 18 opposite directions. That is, in a circumferential direction of the support shaft 15, the first path 20 and the second path 21 of the power transmission member 19 are fixed to an outer circumference of the first treatment pulley 17 and an outer circumference of the second treatment pulley 18, which form a part of an outer circumference of the support shaft 15, so as to face opposite directions.

The first path 20 of the power transmission member 19 is supported on an outer surface of the distal first circumferential portion 7a, extends to intersect a line segment connecting the first rotation axis 4a and the second rotation axis 5a when viewed in the direction in which the first rotation axis 4a and the second rotation axis 5a extend, supported on an outer surface of the proximal first circumferential portion 6a, and extends to the drive control unit 22 through an inside of the long member 2.

The second path 21 of the power transmission member 19 is supported on an outer surface of the distal second circumferential portion 7b, extends to intersect in a direction opposite to the first path 20 with respect to the line segment connecting the first rotation axis 4a and the second rotation axis 5a when viewed in the direction in which the first rotation axis 4a and the second rotation axis 5a extend, is supported on an outer surface of the proximal second circumferential portion 6b, and extends to the drive control unit 22 through the inside of the long member 2.

When viewed in the direction in which the first rotation axis 4a and the second rotation axis 5a extend, the first path 20 and the second path 21 intersect at a point between the first rotation axis 4a and the second rotation axis 5a.

As the treatment piece 16, a known configuration configured to perform treatment on biological tissues, for example, an electrode configured to perform cautery, incision, marking, and other treatments on biological tissues, a puncture needle for puncturing biological tissues, and a suture instrument configured to suture biological tissues, is appropriately selected and applied.

In the present embodiment, the treatment piece 16 is a rod-shaped electrode through which a high frequency current flows. In the present embodiment, the treatment piece 16 is electrically connectable to a high frequency power supply device by a wire (not shown). The treatment piece 16 of the present embodiment has substantially a rod shape whose proximal end is fixed to an intermediate part of the support shaft 15 that extends in a direction perpendicular to the support shaft 15. A distal end of the treatment piece 16 can turn around the center of rotation 15a of the support shaft 15 serving as a turning center. When the treatment portion 14 moves until the distal end of the treatment piece 16 enters the space between the pair of supporting pieces 8a and 8b in the support portion 8, the treatment portion 14 is accommodated into the support portion 8.

The drive control unit 22 is disposed at a proximal end of the long member 2 to perform an operation of rotating the support shaft 15 with respect to the support portion 8 of the link portion 3. The drive control unit 22 includes the driving pulley 23 having an outer circumferential surface around which the intermediate part 19c of the power transmission member 19 hangs, a first power source (not shown) configured to rotate the driving pulley 23, a tension adjusting unit 24 configured to apply tension to the power transmission member 19 by moving the driving pulley 23, and a second power source (not shown) configured to move the bending operation wire 13.

Configurations of the first power source, the second power source, and the tension adjusting unit 24 are not particularly limited. For example, the first power source, the second power source, and the tension adjusting unit 24 may include, for example, a servomotor. Note that the tension adjusting unit 24 may include a biasing member configured to move the driving pulley 23 using a biasing force.

Next, actions of the surgical instrument 1 according to the present embodiment will be described.

In the surgical instrument 1 shown in Fig. 2, the power transmission member 19 fixed to the first treatment pulley 17 and the second treatment pulley 18 of the support shaft 15 hangs on the driving pulley 23 and is pulled to a proximal side by the tension adjusting unit 24 so that tension is applied. In this case, the first path 20 and the second path 21 of the power transmission member 19 are pulled to the proximal side by a pulling force of the same magnitude. In this case, the first path 20 pulls the first treatment pulley 17 to the proximal side, and the second path 21 pulls the second treatment pulley 18 to the proximal side.

Since a force with which the first treatment pulley 17 is pulled in the first path 20 and a force with which the second treatment pulley 18 is pulled in the second path 21 are equal to each other, the support shaft 15 at which the first treatment pulley 17 and the second treatment pulley 18 are provided does not rotate. Further, a force pulling the first treatment pulley 17 and the second treatment pulley 18 to the proximal side does not become a force rotating the first rotary shaft 4 and the second rotary shaft 5 about each rotation axis but is canceled since the power transmission member 19 intersects at a point between the first rotation axis 4a and the second rotation axis 5a. Therefore, in a process in which tension is applied to the power transmission member 19, the treatment portion 14 and the link portion 3 have the same positional relation as before tension is applied without movement.

After tension is applied, by rotating the driving pulley 23, for example, pulling is performed in the first path 20 and a pulling force is released in the second path 21. Therefore, the first treatment pulley 17 is moved to the proximal side, and the support shaft 15 is rotated. For example, pulling is performed in the second path 21, and a pulling force is released in the first path 20. Therefore, the second treatment pulley 18 is moved to the proximal side, and thus the support shaft 15 can be rotated in reverse.

As described above, in the surgical instrument 1 according to the present embodiment, even when the tension is applied to the power transmission member 19 to rotate the treatment portion 14, since a force applying tension is canceled for the link portion 3 configured to swing the treatment portion 14, a force bending the link portion 3 does not act. Therefore, the surgical instrument 1 is easily assembled while appropriate tension is applied.

While the tension is applied to the power transmission member 19, since tension applied to the first treatment pulley 17 and the second treatment pulley 18 is in an equilibrium state, it is possible to reduce an influence of the tension applied to the power transmission member 19 on a bending movement of the link portion 3.

In the above embodiment, an example in which the long member 2 is flexible has been described. However, even when the long member 2 is rigid, it is possible to easily assemble the surgical instrument 1, and an effect in which it is possible to reduce an influence of the tension applied to the power transmission member 19 on a bending movement of the link portion 3 is obtained.

In the surgical instrument 1 according to the present embodiment, while tension applied to the first treatment pulley 17 and the second treatment pulley 18 is in an equilibrium state, tension is applied and a bending movement of the link portion 3 is performed by the power transmission member 19 having two paths. Therefore, the surgical instrument 1 according to the present embodiment has a distal portion whose configuration is compact.

In the above embodiment, the driving pulley 23 and the tension adjusting unit 24 may be detachable. In this case, while the intermediate part 19c of the power transmission member 19 hangs on the driving pulley 23, the driving pulley 23 is removed from the tension adjusting unit 24, tension applied to the treatment portion 14 is thus released, and an operation of replacing the surgical instrument 1 with another surgical instrument can be easily performed. Here, whenever the surgical instrument is replaced, it is necessary to detach the driving pulley 23 and the tension adjusting unit 24. However, in the surgical instrument 1 described in the above embodiment, without complicated adjustment when tension is applied after the driving pulley 23 is attached to the tension adjusting unit 24, it is possible to apply appropriate tension without causing a movement such as unnecessary bending in the treatment portion 14 and the link portion 3.

### (Modification)

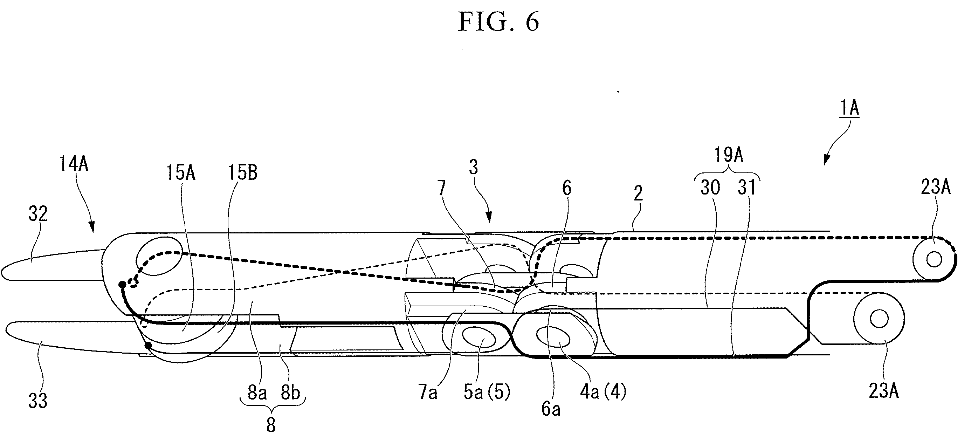

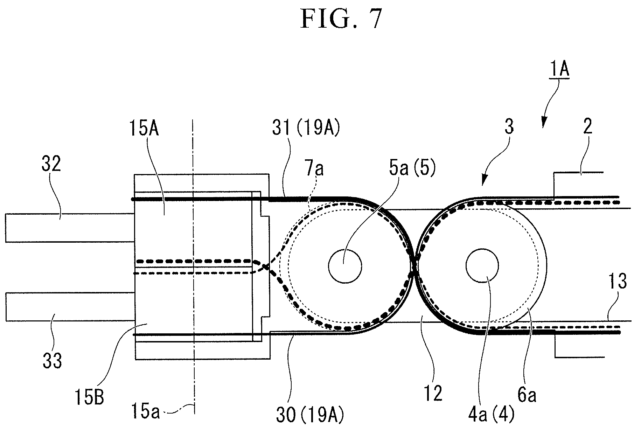

Next, a modification of the above first embodiment will be described. Note that, in the present modification, the same components as in the first embodiment are denoted with the same reference symbols, and repeated descriptions will be omitted. Fig. 6 is a diagram schematically showing a configuration of the present modification. Fig. 7 is a right side view schematically showing a part of a surgical instrument of the present modification.

As shown in Figs. 6 and 7, the surgical instrument 1A of the present modification includes a treatment portion 14A whose configuration is different from the treatment portion 14 described in the first embodiment, and a power transmission member 19A whose configuration is different from the power transmission member 19 described in the first embodiment.

In the present modification, instead of the driving pulley 23 described in the first embodiment, a pair of driving pulleys 23A on which a first wire 30 and a second wire 31 of the power transmission member 19 separately hang are provided. The pair of driving pulleys 23A are rotated independently or cooperatively by, for example, a servomotor.

The treatment portion 14A includes a pair of coaxial support shafts 15A and 15B, and gripping members 32 and 33 that are each fixed to one of the pair of support shafts 15A and 15B, respectively.

In the present modification, when tension is applied to the first wire 30 and the second wire 31, since, like the first embodiment, the treatment portion 14A and the link portion 3 do not move, it is easily assembled, similarly to the above embodiment.

Further, in the present modification, independent power of two systems is transmitted to the treatment portion 14A using a portion through which the power transmission member 19 in the first embodiment does not pass, and it is possible to open and close the gripping members 32 and 33.

While the embodiments of the present invention have been described above in detail with reference to the drawings, a specific configuration is not limited to the embodiments, but also includes design changes without departing from the spirit and scope of the present invention.

For example, instead of a configuration in which the tension adjusting unit 24 pulls the driving pulley 23, a rack to which the power transmission member 19 is fixed is moved by rotation of a pinion, and thus tension may be applied to the power transmission member 19.

In the first embodiment, an example in which the center of rotation 15a of the support shaft 15 is a straight line that extends in a direction perpendicular to the first rotation axis 4a and the second rotation axis 5a is described. However, the center of rotation 15a of the support shaft 15 may extend in a direction intersecting the first rotation axis 4a and the second rotation axis 5a.

Diameters of the first treatment pulley 17 and the second treatment pulley 18 may be different.

When viewed in the direction in which the second rotation axis 5a extends, in the section from the center of rotation 15a of the support shaft 15 to the second rotation axis 5a, it is not necessary for the first path 20 and the second path 21 to be exactly parallel. When viewed in the direction in which the second rotation axis 5a extends, in the section from the center of rotation 15a of the support shaft 15 to the second rotation axis 5a, the first path 20 and the second path 21 may be substantially parallel.

While the embodiments of the present invention have been described above, the scope of the present invention is not limited to the above embodiments. Without departing from the spirit and scope of the present invention, a combination of components in each embodiment can be changed, and the components can be variously changed and deleted. The present invention is not limited to the above descriptions.

### [Industrial Applicability]

It is possible to provide a surgical instrument and a medical manipulator system that are easily assembled.

### [Reference Signs List]

1 Surgical instrument
2 Long member
2a Longitudinal axis
3 Link portion
4 First rotary shaft
4a First rotation axis
5 Second rotary shaft
5a Second rotation axis
8 Support portion
9 Link main body
14, 14A Treatment portion
15, 15A, 15B Support shaft
15a Center of rotation
17 First treatment pulley (first pulley)
18 Second treatment pulley (second pulley)
19, 19A Power transmission member
19a First end
19b Second end
19c Intermediate part
20 First path
21 Second path
22 Drive control unit
23, 23A Driving pulley (moving body)
24 Tension adjusting unit
200a, 200b, 200c, 200d Slave arm
240a, 240b, 240c Surgical instrument
1000 Medical manipulator system

## Claims

1. A surgical instrument comprising:
a long member having a longitudinal axis;
a link portion that is disposed at a distal end of the long member and bendable with respect to the long member in a direction perpendicular to the longitudinal axis;
a treatment portion that is disposed at a distal end of the link portion, includes a shaft that is rotatable with respect to the link portion, and performs treatment on a treatment target region;
a drive control unit disposed at a proximal end of the long member to perform an operation of rotating the shaft with respect to the link portion; and
a power transmission member that is connected to the shaft and the drive control unit and includes a first path and a second path through which power is transmitted from the drive control unit to the shaft,
wherein the link portion includes
a first rotary shaft having a first rotation axis perpendicular to the longitudinal axis at the distal end of the long member, and
a second rotary shaft that is movable with respect to the first rotation axis using the first rotation axis as a center of rotational movement, that has a second rotation axis that extends in parallel with the first rotation axis, and that is linked to the shaft, and
wherein the power transmission member is fixed to a part of an outer circumference of the shaft such that the first path and the second path face opposite directions in a circumferential direction of the shaft, and when viewed in a direction in which the first rotation axis and the second rotation axis extend, the first path and the second path intersect at a point between the first rotation axis and the second rotation axis.

2. The surgical instrument according to claim 1,
wherein, when viewed in a direction perpendicular to the longitudinal axis and perpendicular to the first rotation axis and the second rotation axis, the first path and the second path are spaced from each other in a direction in which the first rotation axis and the second rotation axis extend, in at least a section from a center of rotation of the shaft to the first rotation axis.

3. The surgical instrument according to claim 2,
wherein the shaft includes:
a first pulley at which the first path of the power transmission member is disposed; and
a second pulley at which the second path of the power transmission member is disposed;
wherein the first pulley and the second pulley are spaced from each other in a direction in which the center of rotation of the shaft extends; and
wherein, when viewed in a direction of the second rotation axis, in a section from the center of rotation of the shaft to the second rotation axis, the first path and the second path are substantially parallel.

4. The surgical instrument according to claim 1,
wherein the power transmission member includes a first end and a second end fixed to the shaft, and an intermediate part disposed at the drive control unit; and
wherein the drive control unit includes:
a moving body linked to the intermediate part; and
a tension adjusting unit capable of applying tension to the power
transmission member by moving the moving body.

5. A medical manipulator system comprising:
the surgical instrument according to any one of claims 1 to 4; and
an arm attached to the surgical instrument and configured to move the surgical instrument.
